(19) Europäisches Patentamt
European Patent Office
Office européen des brevets

(11) Veröffentlichungsnummer: **0 176 035**
**B1**

(12) # EUROPÄISCHE PATENTSCHRIFT

(45) Veröffentlichungstag der Patentschrift: **06.06.90**

(21) Anmeldenummer: **85111840.6**

(22) Anmeldetag: **19.09.85**

(51) Int. Cl.[5]: **C 07 C 65/28,** C 07 C 255/50, C 07 C 69/76, C 07 C 69/14, C 07 C 43/215, C 07 C 43/23, C 07 C 47/575, C 07 C 57/50, C 07 C 211/26, C 07 C 233/00, C 07 C 239/06

(54) **1-substituierte Tetralinderivate, ihre Herstellung und Verwendung.**

(30) Priorität: **22.09.84 DE 3434944**

(43) Veröffentlichungstag der Anmeldung:
**02.04.86 Patentblatt 86/14**

(45) Bekanntmachung des Hinweises auf die Patenterteilung:
**06.06.90 Patentblatt 90/23**

(84) Benannte Vertragsstaaten:
**AT BE CH DE FR GB IT LI NL SE**

(56) Entgegenhaltungen:
**EP-A-0 002 742**
**EP-A-0 084 667**
**EP-A-0 176 875**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Wuest, Hans-Heiner, Dr.**
**Unteres Bieth 10**
**D-6901 Dossenheim (DE)**
Erfinder: **Frickel, Fritz-Frieder, Dr.**
**Silvanerweg 7**
**D-6705 Deidesheim (DE)**
Erfinder: **Nuerrenbach, Axel, Dr.**
**Koenigsberger Strasse 7**
**D-6718 Gruenstadt (DE)**

(56) Entgegenhaltungen:
**EUROPEAN JOURNAL OF MEDICINAL CHEMISTRY - CHIMICA THERAPEUTICA, Band XV, Nr. 1, Januar-Februar 1980, Seiten 9-15; P. LOELIGER et al.: "Arotinoids, a new class of highly active retinoids"**

**EP 0 176 035 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Es ist bekannt (DE—OS 2 854 354, DE—OS 3 202 118, Eur. J. Med. Chem.-Chimica Therapeutica *15* (1980), 9—15), daß Stilben-Derivate pharmakologische Wirkungen bei der topischen und systemischen Therapie von Neoplasien, Akne, Psoriasis und anderen dermatologischen Affektionen aufweisen. Deren Wirkung ist jedoch nicht immer befriedigend, insbesondere in Relation zu den Nebenwirkungen.

Es wurde nun gefunden, daß 1-substituierte Tetraline der Formel I

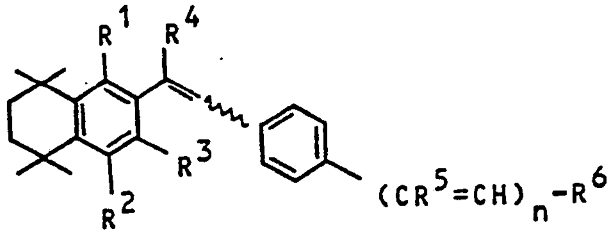

I,

in der

n die Zahl 0 oder 1,

$R^1$ eine Hydroxy- oder $C_{1-6}$-Alkoxygruppe,

$R^2$ und $R^3$ Wasserstoff- oder Halogenatome oder $C_{1-4}$-Alkyl- oder Methoxygruppen,

$R^4$ ein Wasserstoffatom oder eine gegebenenfalls cyclische Alkylgruppe mit bis zu 6 Kohlenstoffatomen,

$R^5$ ein Wasserstoffatom oder eine $C_{1-4}$-Alkylgruppe,

$R^6$ ein Wasserstoffatom, eine Nitril- oder $C_{2-10}$-Ketalgruppe, einen 2-Oxazolinyl- oder Tetrazolylrest oder den Rest —$CHR^7R^8$ oder —CO—$R^9$ bedeuten, worin

$R^7$ ein Wasserstoffatom oder eine $C_{1-3}$-Alkylgruppe

$R^8$ ein Wasserstoffatom oder den Rest —$OR^{10}$ oder —$NR^{11}R^{12}$ (mit $R^{10}$ in der Bedeutung eines Wasserstoffatoms, einer $C_{1-4}$-Alkyl-, $C_{1-20}$-Alkanoyl-, gegebenenfalls substituierten Aralkyl- oder gegebenenfalls substituierten Benzoylgruppe und $R^{11}$ und $R^{12}$ in der Bedeutung von Wasserstoffatomen, $C_{1-4}$-Alkyl-, $C_{1-20}$-Alkanoyl- oder gegebenenfalls substituierten Benzylgruppen),

$R^9$ ein Wasserstoff- oder Halogenatom, eine $C_{1-4}$-Alkylgruppe, den Azido-, Imidazol- oder Thiazolrest oder den Rest —$OR^{13}$ oder —$NR^{14}R^{15}$ darstellen (mit $R^{13}$ in der Bedeutung eines Wasserstoffatoms, einer gegebenenfalls durch Hydroxygruppen oder eine $C_{1-6}$-Alkoxygruppe substituierten $C_{1-8}$-Alkylgruppe, einer gegebenenfalls substituierten Aryl- oder Aralkylgruppe und mit $R^{14}$ und $R^{15}$ in der Bedeutung von Wasserstoffatomen, $C_{1-6}$-Alkyl- oder gegebenenfalls substituierten Aryl- oder Aralkylgruppen oder Tetrazolylreste),

sowie deren physiologisch verträglichen Salze ein besseres Wirkungsspektrum und einen besseren therapeutischen Index besitzen.

Als Arylgruppe ist die Phenylgruppe bevorzugt, die mit einer Methyl-, Methoxy- oder Nitrogruppe substituiert sein kann; als Aralkylgruppe ist die Benzylgruppe bevorzugt, die im Arylteil mit einer Methyl- oder Methoxygruppe oder mit Halogen substituiert sein kann. Substituenten der Benzoylgruppe können beispielsweise die Methyl- oder die Methoxygruppe oder Halogen sein. Als Halogenatome sind für $R^2$ und $R^3$ vorzugsweise Fluor und für $R^{10}$ vorzugsweise Fluor oder Chlor zu nennen.

Typische Beispiele für erfindungsgemäße Verbindungen sind:

4-[2-(5,6,7,8-Tetrahydro-1-methoxy-3,5,5,8,8-tetramethyl-2-naphthyl)-1-propylen]benzoesäure
4-[2-(5,6,7,8-Tetrahydro-1,3-dimethoxy-5,5,8,8-tetramethyl-1-naphthyl)-1-propylen]benzoesäure
4-[2-(5,6,7,8-Tetrahydro-1-methoxy-4,5,5,8,8-pentamethyl-2-naphthyl)-1-propenyl]benzoesäure
4-[2-(5,6,7,8-Tetrahydro-1,4-dimethoxy-5,5,8,8-tetramethyl-2-naphthyl)-1-propenyl]benzoesäure
4-[2-(5,6,7,8-Tetrahydro-1-hydroxy-3,5,5,8,8-pentamethyl-2-naphthyl)-1-propenyl]benzoesäure
4-[2-(1-Hexyloxy-5,6,7,8-tetrahydro-3,5,5,8,8-pentamethyl-2-naphthyl)-1-propenyl]benzoesäure
4-[2-(1-Ethoxy-5,6,7,8-tetrahydro-3,5,5,8,8-pentamethyl-2-naphthyl)-1-propenyl]benzoesäure
4-[2-(5,6,7,8-Tetrahydro-1,4-dimethoxy-3,5,5,8,8-pentamethyl-2-naphthyl)-1-propenyl]benzoesäure
4-[2-(3-Fluor-5,6,7,8-tetrahydro-1-methoxy-5,5,8,8-tetramethyl-2-naphthyl)-1-propenyl]benzoesäure
4-[2-(3-Chlor-5,6,7,8-tetrahydro-1-methoxy-5,5,8,8-tetramethyl-2-naphthyl)-1-propenyl]benzoesäure
4-[2-(5,6,7,8-Tetrahydro-1,3-dimethoxy-4,5,5,8,8-pentamethyl-2-naphthyl)-1-propenyl]benzoesäure
4-[2-(5,6,7,8-Tetrahydro-1-methoxy-3,5,5,8,8-pentamethyl-2-naphthyl)-1-ethenyl]benzoesäure
4-[2-(5,6,7,8-Tetrahydro-1,3-dimethoxy-5,5,8,8-tetramethyl-2-naphthyl)-1-ethenyl]benzoesäure
4-[2-(5,6,7,8-Tetrahydro-1,4-dimethoxy-5,5,8,8-tetramethyl-2-naphthyl)-1-ethenyl]benzoesäure
4-[2-(5,6,7,8-Tetrahydro-1-methoxy-4,5,5,8,8-pentamethyl-2-naphthyl)-1-ethenyl]benzoesäure
4-[2-(5,6,7,8-Tetrahydro-1-hydroxy-3,5,5,8,8-pentamethyl-2-naphthyl)-1-ethenyl]benzoesäure
4-[2-(1-Hexyloxy-5,6,7,8-tetrahydro-3,5,5,8,8-pentamethyl-2-naphthyl)-1-ethenyl]benzoesäure
4-[2-(1-Ethoxy-5,6,7,8-tetrahydro-3,5,5,8,8-pentamethyl-2-naphthyl)-1-ethenyl]benzoesäure
4-[2-(5,6,7,8-Tetrahydro-1,4-dimethoxy-3,5,5,8,8-pentamethyl-2-naphthyl)-1-ethenyl]benzoesäure

4-[2-(3-Fluor-5,6,7,8-tetrahydro-1-methoxy-5,5,8,8-tetramethyl-2-naphthyl)-1-ethenyl]benzoesäure
4-[2-(5,6,7,8-Tetrahydro-1-methoxy-3,5,5,8,8-pentamethyl-2-naphthyl)-1-buten(1)-yl]benzoesäure
4-[2-(5,6,7,8-Tetrahydro-1,3-dimethoxy-5,5,8,8-tetramethyl-2-naphthyl)-1-buten(1)-yl]benzoesäure
4-[2-(5,6,7,8-Tetrahydro-1,4-dimethoxy-5,5,8,8-tetramethyl-2-naphthyl)-1-buten(1)-yl]benzoesäure
4-[3-Methyl-2-(5,6,7,8-tetrahydro-1-methoxy-3,5,5,8,8-pentamethyl-2-naphthyl)-1-buten(1)-yl]-benzoesäure
4-[2-Cyclopropyl-2-(5,6,7,8-tetrahydro-1-methoxy-3,5,5,8,8-pentamethyl-2-naphthyl)-1-ethenyl]-benzoesäure
4-[2-(5,6,7,8-Tetrahydro-1-methoxy-3,5,5,8,8-pentamethyl-2-naphthyl)-1-hexen(1)-yl]benzoesäure
4-[2-Cyclohexyl-2-(5,6,7,8-tetrahydro-1-methoxy-3,5,5,8,8-pentamethyl-2-naphthyl)-1-ethenyl]-benzoesäure.

In diesen Verbindungen sind anstelle der Carboxylgruppe folgende Reste außerdem typisch:

Methoxycarbonyl-, Ethoxycarbonyl-, Propyloxycarbonyl-, Butoxycarbonyl-, Benzyloxycarbonyl-, Azidocarboncyl-, Chlorcarbonyl-, Fluorcarbonyl-, Cyano-, Formyl-, Hydroxymethyl-, Methyl-, Acetyl-, Methoxmethyl-, Ethoxymethyl-, Benzyloxymethyl-, Formyloxymethyl-, Acetoxymethyl-, Propionyloxymethyl-, Hexadecanoyloxymethyl-, Benzyloxymethyl-, (3,4-Dimethoxy)benzyloxymethyl-, Aminomethyl-, Methylaminomethyl-, Ethylaminomethyl-, Propylaminomethyl-, Butylaminomethyl-, Acetylaminomethyl-, Formylaminomethyl-, Benzoylaminomethyl-, (4-Methoxy)benzoylaminomethyl-, Dimethylaminomethyl-, Morpholinmethyl-, Pyrrolidinomethyl-, Piperidinomethyl-, Oxazolin-2-yl-, Dimethoxymethyl-, (E)-2-Carbethoxyethenyl-, (E)-2-Carboxyethenyl-, Wasserstoff, Carbamoyl-, Methylcarbamoyl-, Dimethylcarbamoyl-, Morpholinocarbamoyl-, Benzylcarbamoyl, Phenylcarbamoyl-, Tetrazolyl.

Die erfindungsgemäßen Verbindungen kann man auf verschiedenen Wegen herstellen, die im Prinzip jeweils bekannt sind. Z.B. kann man eine Carbonylverbindung der Formel II

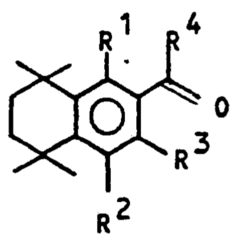

II,

in der $R^1$, $R^2$, $R^3$ und $R^4$ die angegebenen Bedeutungen haben, mit einer Phosphorverbindung der Formel III

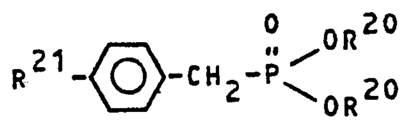

III,

in der $R^{21}$ ein Wasserstoffatom, eine $C_{1-4}$-Alkylgruppe die Nitrilgruppe oder eine Gruppe —$COOR^{22}$, und $R^{20}$ und $R^{22}$ eine $C_{1-3}$-Alkylgruppe bedeutet, nach Wittig-Horner umsetzen. Zweckmäßigerweise arbeitet man in einem Lösungsmittel in Gegenwart der für Wittig-Horner-Reaktionen üblichen basischen Verbindungen.

Die Umsetzung nach Wittig-Horner verläuft bei einer Temperatur von bis zu 100°C, zweckmäßig bei 20 bis 50°C. Die Umsetzung kann bei atmosphärischem Druck oder in einem geschlossenen Gefäß unter erhöhtem Druck, gegebenenfalls unter Erwärmung auf den angegebenen Temperaturbereich durchgeführt werden.

Man kann diese Umsetzung in Gegenwart eines Verdünnungs- oder Lösungsmittels, beispielsweise eines niederen gesättigten Dialkylethers, Dialkylglykolethers oder cyclischen Ethers, die Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchführen. Bevorzugt verwendet man cyclische Ether, wie Dioxan oder Tetrahydrofuran sowie insbesondere Dimethylformamid oder deren Mischungen, wobei die Umsetzung im allgemeinen bei einer Temperatur bis zu 30°C abläuft.

Die Umsetzungen werden in Gegenwart eines Deprotonierungsmittels für das Phosphat (III) vorgenommen. Geeignet sind Alkalimetallhydride und Alkalimetallamide, insbesondere des Natriums und Kaliums, die Natrium- und Kaliumsalze von Dimethylsulfoxid, Alkyllithiumverbindungen wie n-Butyllithium oder Alkalimetallalkoholate, vorzugsweise Natriummethanolat und Natriumethanolat.

Man kann die erfindungsgemäßen Verbindungen auch durch Wittig-Reaktion eines Phosphoniumsalzes der Formel IV

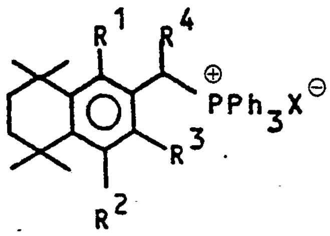

IV,

in der $R^1$, $R^2$, $R^3$ und $R^4$ die oben angegebenen Bedeutungen haben und $X^{\ominus}$ für ein Anion, vorzugsweise Chlor oder Brom, steht, mit einem p-Carbalkoxy-benzaldehyd der Formel V

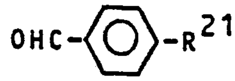

V,

wobei $R^{21}$ die genannte Bedeutung hat, erhalten.

Die Wittig- oder Wittig-Horner-Reaktion liefert ülicherweise Gemische der sterisch isomeren (E/Z)-Olefine.

E/Z-Isomerengemische mit überwiegendem Z-Anteil werden unter Lichteinwirkung an der olefinischen Doppelbindung zu Gemischen mit höherem Anteil der (E)-Isomeren umgelagert. Aus den erhaltenen (E/Z)-Isomerengemischen mit jetzt günstigerem (E)-Gehalt werden vorteilhaft reine (E)-Verbindungen der Formel (I), vorzugsweise durch Kristallisation oder eine chromatographische Methode wie die Säulen- oder präparative HPL-Chromatographie erhalten.

Die Photoisomerisierung wird vorzugsweise in Lösung durchgeführt. Geeignete Lösungsmittel sind polare protische und aprotische Lösungsmittel z.B. Methanol, Ethanol, Essigester, Tetrahydrofuran, Aceton. Die Konzentration der bestrahlten Lösung liegt bei 0,1 bis 50, vorzugsweise bei 1 bis 15 Gewichtsprozent.

Man kann in Gegenwart von Sensibilisatoren wie beispielsweise Acetophenon, 4-Methoxyacetophenon, Propiophenon, Benzol, Aceton, Benzophenon, Benzil, Michlers Keton bestrahlen. Besonders bevorzugt ist hier Aceton.

Als Lichtquelle zur Durchführung der genannten Photoreaktion können künstliche Strahler, deren Emission zumindest teilweise im Bereich von 200 bis 600 nm, vorzugsweise im Bereich von 300 bis 400 nm liegt, verwendet werden. Vorteilhaft sind Quecksilberdampf-, Fluor-, Xenon- oder Wolframlampen, Leuchtstoffröhren oder Kohlebogenlampen.

Die Bestrahlungstemperatur ist abhängig von der Art des verwendeten Lösungsmittels. Besonders bevorzugt ist der Bereich von +10 bis +30°C. Die Strahlungswärme kann durch Lampenkühlung und/oder Kühlung des Reaktionsgemisches abgeführt werden, wobei in Lampenkühlkreis destilliertes Wasser oder in bekannter Weise mit Zusätzen versehene, filternde Lösungen eingesetzt werden können.

Die für die Wittig-Horner-Reaktion benötigten Ketone und Aldehyde der Formel II lassen sich beispielsweise durch Acylierung der entsprechenden Tetrahydrotetramethylnaphthaline in Gegenwart einer Lewis-Säure herstellen. Besonders geeignet als Acylierungsmittel sind Acylhalogenide, vornehmlich die Acylchloride. Bevorzugte Lewis-Säuren sind Eisen(III)chlorid, Aluminium(III)chlorid und Titan(IV)-chlorid. Die Formylierung kann vorteilhaft mit Hexamethylentetramin/Trifluoressigsäure durchgeführt werden. Die Tetrahydrotetramethylnaphthaline sind in US—PS 3 442 640 und US—PS 3 499 751 beschrieben oder können nach dem dort beschriebenen Verfahren aus 2,5-Dichlor-2,5-dimethylhexan und einem entsprechend substituierten Benzol durch Friedel-Crafts-Alkylierung hergestellt werden.

Die Phosphoniumsalze der Formel IV können wie folgt erhalten werden: Eine Carbonylverbindung der Formel II wird zunächst mittels eines komplexen Metallhydrides wie Natriumborhydrid oder Lithiumaluminiumhydrid zum entsprechenden Alkohol reduziert. Dieser wird mit einem Phosphorhalogenid, wie Phosphortribromid oder Phosphoroxychlorid, in Gegenwart einer Base wie Pyridin halogeniert. Nachfolgende Umsetzung mit Triphenylphosphin liefert das Phosphoniumsalz der Formel IV.

Die Benzoesäureester der allgemeinen Formel I, in denen n = 0 und $R^6$ eine Carboalkoxygruppe bedeuten, werden, falls dies erwünscht ist, in die freien Carbonsäuren und ihre physiologisch verträglichen Salze, durch Esterverseifung überführt Umgekehrt läßt sich natürlich die freie Säure in bekannter Weise verestern.

Zweckmäßigerweise wird die Verseifung/Veresterung in Gegenwart eines Verdünnungs- oder Lösungsmittels, beispielsweise eines Dialkylglykolethers oder cyclischen Ethers, wie 1,2-Dimethoxyethan, Tetrahydrofuran oder Dioxan, eines niederen aliphatischen Ketons, wie Aceton, Methylethylketon oder Methylisobutylketon, oder in einem niederen aliphatischen Alkohol, wie Methanol, Ethanol, Propanol oder

Isopropanol, gegebenenfalls in Gegenwart von Wasser bzw. in Mischungen der genannten Lösungsmittel mit Wasser durchgeführt.

Bevorzugte Lösungsmittel sind wäßrige Mischungen von Ethanol und Methanol, wobei die Umsetzung beim Siedepunkt des Reaktionsgemisches durchgeführt wird.

Die Verseifung geschieht bevorzugt in Gegenwart von Alkali, wie Alkalimetallhydroxide, Alkalimetallcarbonate und -hygrogencarbonate, insbesondere des Natriums und Kaliums, organischen tertiären Basen, wie Pyridin oder niedere Trialkylamine, wie Trimethyl- oder Triethylamin in Gemischen mit Wasser. Dabei wird die verwendete Base im Verhältnis zum Ester in stöchiometrischer Menge oder in geringem Überschuß eingesetzt. Vorzugsweise wird Natrium- oder Kaliumhydroxid verwendet.

Die erfindungsgemäßen Amide können in an sich bekannter Weise hergestellt werden, indem man die entsprechenden Benzoesäuren zunächst in carbonylaktivere Derivate überführt, z.B. in die Säurehalogenide, -azide, -imidazolide, oder -anhydride, die O-Acyl-N,N'-dicyclohexylisoharnstoffe oder p-Nitrophenylester, und diese mit Aminen $HNR^{11}R^{12}$ behandelt. In den Fällen besonders reaktiver Amine, vor allem Ammoniak, ist die direkte Amidolyse von Estern (mit dem Rest $-OR^{10}$) bevorzugt.

Ein Halogenid einer Carbonsäure, vorzugsweise das Säurechlorid, kann durch Reaktion mit 2-Aminoethanol und anschließende Cyclisierung in ein Oxazolinderivat der Formel I überführt werden.

Eine Carbonsaure, ein Carbonsäureester oder ein Carbonsäureamid der Formel I kann in an sich bekannter Weise zu den entsprechenden Alkoholen bzw. Aminen reduziert werden. Vorteilhaft wird die Reduktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminiumhydrid oder Diisobutylaluminiumhydrid eingesetzt. Als Lösungsmittel werden beim Arbeiten mit Lithiumaluminiumhydrid Ether eingesetzt, wie Diethylether, Dioxan oder Tetrahydrofuran. Führt man die Reduktion mit Diisobutylaluminiumhydrid oder einem Alkoxynatriumaluminiumhydrid durch, so ist die Verwendung von Kohlenwasserstoffen wie Hexan oder Toluol bevorzugt.

Ein Amin oder ein Alkohol der Formel I kann in an sich bekannter Weise mit einem Alkanoylhalogenid oder -anhydrid, einem Aralkylhalogenid oder -anhydrid oder einem Aroyl- oder Heteroaroylhalogenid oder -anhydrid zweckmäßigerweise in einem inerten Verdünnungs- oder LÖsungsmittel, z.B. einem niederen aliphatischen Keton wie Aceton, Methylethylketon oder Methylisobutylketon, einen Dialkylformamid wie Dimethylformamid oder Diethylformamid oder mit überschüssigem Acylierungsmittel als Verdünnungs- oder Lösungsmittel in die erfindungsgemäßen Amide und Ester überführt werden. Bevorzugt werden die Umsetzung in Gegenwart einer Base als säurebindendes Mittel in einem zwischen $-20°C$ und dem Siedepunkt des Reaktionsgemisches liegenden Temperaturbereich vorgenommen. Geeignete Basen sind Alkalimetallcarbonate, -hydrogencarbonate, -hydroxide oder -alkoholate, insbesondere des Natriums und Kaliums, basische Oxide, wie Aluminiumoxid oder Calciumoxid, organische tertiäre Basen, wie Pyridin, oder niedere Trialkylamine, wie Trimethyl- oder Triethylamin. Dabei können die Basen im Verhältnis zum eingesetzten Alkylierungsmittel in katalyatischer Menge oder in stöchiometrischer Menge bzw. in geringem Überschuß verwendet werden.

Ein Alkohol der Formel I kann mit Alkylhalogeniden $R^{12}$-J, $R^{12}$-Br oder $R^{12}$-Cl in Gegenwart von Alkalimetallhydriden, vorzugsweise Natriumhydrid oder in Gegenwart von Alkyllithium-Verbindungen, vorzugsweise n-Butyllithium, in einem organischen Lösungsmittel wie Tetrahydrofuran, Dioxan, 1,2-Dimethoxyethan, Methyl-tert.-butylether oder bei Verwendung von Natriumhydrid auch in Dimethylformamid in einem zwischen $-10°C$ und $40°C$ liegenden Temperaturbereich zu einem Ether der Formel I umgesetzt werden.

Ein Alkohol der Formel I kann mit geeigneten Oxidationsmitteln, vorzugsweise Mangan(IV)-oxid, gegebenenfalls auf einem anorganischen Trägermaterial wie Silicagel oder Aluminiumoxid zu einem Aldehyd der Formel I oxidiert werden. Vorteilhaft arbeitet man in einem inerten organischen Lösungsmittel, beispielsweise einem Kohlenwasserstoff wie Hexan oder in einem Ether wie beispielsweise Tetrahydrofuran oder in Mischungen der genannten Lösungs- und Verdünnungsmittel im Temperaturbereich zwischen $-10°C$ und $30°C$. Die benötigte Reaktionszeit ist im wesentlichen von der Oxidationsaktivität des eingesetzten Mangan(IV)-oxids abhängig.

Einen Aldehyd der Formel I kann man auch durch Reduktion des entsprechenden Nitrils der Formel I mit Diisobutylaluminiumhydrid in einem Lösungsmittel vorzugsweise in Toluol, Hexan, Tetrahydrofuran oder Mischungen dieser Lösungsmittel in einem Temperaturbereich zwischen $-40°C$ und Raumtemperatur erhalten.

Eine Carbonylverbindung der Formel I (im Fall n = 0) kann mit einer Phosphorverbindung der Formel VI oder VII

$$(R^{20}O)_2\overset{\overset{\displaystyle O}{\|}}{P}CH_2CN \qquad (R^{20}O)_2\overset{\overset{\displaystyle O}{\|}}{P}CH_2COOR^{20}$$

VI VII,

in der $R^{20}$ die angegebene Bedeutung hat, in einer Wittig-Horner-Reaktion umgesetzt werden, zweckmäßigerweise arbeitet man in einem Lösungsmittel, vorzugsweise Tetrahydrofuran, Dimethylformamid oder Dimethylsulfoxid, und in Gegenwart der für solche Olefinierungen üblichen Basen wie z.B. Natriumhydrid oder Natriumethanolat. Die Umsetzung verläuft bei einer Temperatur bis zu 100°C, zweckmäßig bei 20 bis 50°C.

Die Nitril- bzw. Estergruppe wird anschließend, falls erwünscht, nach den vor- und nachstehend beschriebenen Methoden in andere funktionelle Gruppen umgewandelt.

Ein Nitril der Formel I kann in an sich bekannter Weise unter Säure- oder vorteilhafter Basenkatalyse zur entsprechenden Carbonsäure verseift werden. Als Basen bevorzugt sind Alkalimetallhydroxide, besonders Kaliumhydroxid, das im Überschuß eingesetzt wird. Als Lösungsmittel werden in der Regel mit Wasser mischbare Alkohole wie z.B. Methanol, Ethanol, Isopropanol oder n-Butanol eingesetzt. Die Umsetzung wird üblicherweise beim Siedepunkt des Reaktionsgemisches durchgeführt.

Aus den Nitrilen der Formel I können durch Addition eines Azids, z.B. eines Alkalimetallazids, vorzugsweise Natriumazid, in Gegenwart von Aluminiumchlorid oder Ammoniumchlorid die entsprechenden Tetrazole der Formel I erhalten werden. Als Lösungsmittel verwendet man bevorzugt cyclische Ether, wie Dioxan oder Tetrahydrofuran, sowie insbesondere Dimethylformamid oder deren Mischungen, wobei die Umsetzung im allgemeinen bei einer Temperatur von 60—100°C abläuft.

Einige der erfindungsgemäßen Verbindungen weisen ein acides Wasserstoffatom auf und können daher mit Basen in üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z.B. Methylamin, Ethylamin oder Cyclohexylamin, oder mit substituierten niederen Alkylaminen, insbesondere hydroxysubstituierten Alkylaminen, wie Diethanolamin, Triethanolamin oder Tris-(hydroxymethyl)-aminomethan, sowie mit Piperidin oder Morpholin.

Gegebenenfalls werden die erhaltenen erfindungsgemäßen Amine der Formel (I) nach bekannter Verfahrensweise in das Säureadditionssalz einer physiologisch verträglichen Säure überführt. Als übliche physiologisch verträgliche organische oder anorganische Säure kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band *10*, Seiten 224—225, Birkhäuser Verlag, Basel und Stuttgart, 1966, entnommen werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertreachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel (I) inhibiert wird. Diese Methodik kann aus Cancer Res. *36*, 964—972 (1976) oder aus Nature *250*, 64—66 (1974) und Nature *253*, 47—50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. *60*, 1035—1041 (1978), Esperimental Cell Research *117*, 15—22 (1978) und Proc. Natl. Acad. Sci. USA *77*, 2937—2940 (1980) entnommen werden.

Die antiarthritische Wirkung der erfindungsgemäßen Verbindungen kann in üblicher Weise im Tierexperiment im Adjuvans-Arthritis-Modell bestimmt werden. Die dermatologische Aktivität, beispielsweise für die Behandlung von Akne, kann u. a. durch die komedolytische Aktivität und die Fähigkeit nachgewiesen werden, die Anzahl der Zysten im Modell der Rhino-Maus zu reduzieren.

Diese Methode ist von L. H. Kligman et al in The Journal of Investigative Dermatology *73*, 354—358 (1978) und von J. A. Mezick et al. in "Models of Dermatology", Ed. Maibach, Lowe, Vol. 2, 5.59—63, Karger, Basel 1985, beschrieben.

Die Testsubstanz wurde in einem geeigneten Vehikel auf der gesamten Rückenpartie der Rhino-Maus topisch aufgetragen (100 µl), einmal täglich, an fünf aufeinanderfolgenden Tagen pro Woche, zwei Wochen lang. Ungefähr 72 h nach der letzten Behandlung wurde die dorsale Haut entfernt und in 0,5 %iger Essigsäure 18 h bei 4—6°C belassen. Danach wurde eine ca. $2 \times 5\ cm^2$ große Fläche herausgeschnitten, die Epidermis abgeschält, auf einen Objektträger aufgebracht (mit der dermalen Seite nach oben) und mittels Alkohol/Xylol wasserfrei gespült, bis die Epidermis durchsichtig erscheint. Die Probe wurde durch Überziehen mit Permount fixiert und mikroskopisch ausgewertet. Gemessen wurde der Durchmesser von jeweils 10 Utriculi in jeweils 5 frei gewählten Feldern und daraus durch Vergleich mit der unbehandelten

Kontrollgruppe die durchschnittliche Reduktion des Utriculusdurchmessers berechnet. Die folgende Tabelle zeigt die erhaltenen Ergebnisse.

Tabelle

| Substanz | Dosis mg/ml | Reduktion des Utriculusdurchmessers in % |
|---|---|---|
| Beispiel 17 | 2 | 68,6 |
| | 0,2 | 53,9 |
| Beispiel 19 | 2 | 72,5 |
| | 0,2 | 56,0 |
| Beispiel 16 | 1 | 73,6 |
| | 0,1 | 49,8 |
| Beispiel 14 | 1 | 77,5 |
| | 0,1 | 55,0 |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutischtechnischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1%iger Konzentration, bevorzugt in 0,001 bis 0,1%iger Konzentration und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hydroxyanisol oder butyliertes Hydroxytoluol oder geschmacksverbessernde Zusätze, Stabilisierungsmittel, Emulgiermittel, Gleichmittel usw. zugesetzt werden. Voraussetzung ist, daß alle bei der Herstellung pharmazeutischer Zubereitung verwendeten Stoffe toxikologisch unbedenklich sind und mit den verwendeten Wirkstoffen verträglich sind.

Herstellung der erfindungsgemäßen Verbindungen

A. *Herstellung von Ausgangsverbindungen*

2-Formyl-5,6,7,8-tetrahydro-1-alkoxy-3,5,5,8,8-pentamethylnaphthaline

340 ml Dimethylsulfoxid und 32,2 g Kaliumhydroxid (Plätzchen) werden 5 min gerührt, dann gibt man nacheinander 0,1 mol 2-Formyl-5,6,7,8-tetrahydro-3,5,8,8-pentamethyl-1-naphthol und 0,24 mol Alkylhalogenid zu, wobei die Temperatur etwas ansteigt. Man läßt über Nacht bei Raumtemp. rühren und extrahiert danach mit Ether/Wasser. Die Etherphase wird mehrmals mit Wasser gewaschen, getrocknet ($Na_2SO_4$) und eingeengt. Das so erhaltene Rohprodukt wird, falls nötig, durch Destillation gereinigt.

Nach diesem Verfahren wurden folgende Verbindungen hergestellt:

1-Ethoxy-2-formyl-5,6,7,8-tetrahydro-3,5,5,8,8-pentamethylnaphthalin, Sdp. 130—1323°C (0,2 mbar), Ausbeute 87%.

2-Formyl-5,6,7,8-tetrahydro-3,5,5,8,8-pentamethyl-1-propyloxynaphthalin, Ausbeute 100% (roh).

1-(Methyl)ethoxy-2-formyl-5,6,7,8-tetrahydro-3,5,5,8,8-pentamethylnaphthalin, Sdp. 112—117°C (0,1 mbar), Ausbeute 70%.

1-Butyloxy-2-formyl-5,6,7,8-tetrahydro-3,5,5,8,8-pentamethylnaphthalin, Sdp. 140°C (0,2 mbar), Ausbeute 82%.

2-Formyl-1-hexyloxy-5,6,7,8-tetrahydro-3,5,5,8,8-pentamethylnaphthalin, Ausbeute 100% (roh).

B. *Herstellung der Endprodukte*

Beispiel 1

(E)-4-[2-(5,6,7,8-Tetrahydro-1-methoxy-3,5,5,8,8-pentamethyl-2-naphthyl)-1-ethenyl]benzoesäureethylester

Zu einer Suspension von 25 ml absolutem Dimethylsulfoxid und 0,75 g (25 mmol) Natriumhydrid (80%ig, zuvor mit Petrolether vom 20%igen Paraffinanteil befreit), wurde bei Raumtemperatur beginnend eine Lösung von 7,5 g (25 mmol) 4-Carboxyethylbenzylphosphorsäurediethylester in 12 ml Dimethylsulfoxid zugetropft. Anschließend wurde noch 30 min bei 35 bis 40°C gerührt. Dann wurden innerhalb von 10 min 3 g (12,5 mmol) 2-Formyl-5,6,7,8-tetrahydro-1-methoxy-3,5,5,8,8-pentamethyl-naphthalin, gleöst in 12 ml Dimethylsulfoxid und 1 nl Tetrahydrofuran, zugetgropft. Man ließ 3 h bei Raumtemperatur nachrühren. Danach wurde der Ansatz auf 300 ml Wasser gegossen und mit 2 N Salzsäure angesäuert. Der schmierige Rückstand wurde abgetrennt, mit Methanol verrührt und abfiltriert. Nach säulenchromatographischer Reinigung des Feststoffs (Kieselgel; Toluol) erhielt man 1,9 g der Titelverbindung, Schmp. 123,2°C.

Die HPLC-Analyse (Si 60, 5 µm, 150 bar, n-Heptan/Essigester (97:3), $t_R = 6{,}7$ min) weist eine Isomerenreinheit größer als 98% aus.

Beispiel 2

(E)-4-[2-(5,6,7,8-Tetrahydro-1-methoxy-3,5,5,8,8-pentamethyl-2-naphthyl)-1-ethenyl)]benzonitril

Zu einer Suspension von 3 g (0,1 mol) Natriumhydrid (80%ig, zuvor mit Petrolether vom 20%igen Paraffinanteil befreit), in 100 ml absolutem Dimethylsulfoxid wurde bei 25 bis 40°C eine Lösung von 25,2 g (0,1 mol) 4-Cyanobenzylphosphonsäurediethylester in 50 ml Dimethylsulfoxid zugetropft. Es wurde noch 1 h nachgerührt und dann eine Lösung von 12 g (50 mmol) 2-Formyl-5,6,7,8-tetrahydro-1-methoxy-3,5,5,8,8-pentamethylnaphthalin in 100 ml Dimethylsulfoxid zugetropft. Man ließ über Nacht bei Raumtemperatur rühren. Danach wurde das Reaktionsgemisch auf 1,2 l Wasser gegossen und mit 2 N Salzsäure angesäuert. Das entstandene ölige Produkt wurde abgetrennt, mit wenig Methanol verrührt und das entstandene Kristallisat abfiltriert. Der Feststoff wurde auf dem Filter mit Methanol nachgewaschen. Nach dem Trocknen verblieben 13,8 g der Titelverbindung, Schmp. 120°C.

Beispiel 3

(E)-4-[2-(5,6,7,8-Tetrahydro-1-methoxy-3,5,5,8,8-pentamethyl-2-naphthyl)-1-ethenyl]benzoesäure

5,1 g (15 mmol) (E)-4-[2-(5,6,7,8-Tetrahydro-1-methoxy-3,5,5,8,8-pentamethyl-2-naphthyl)-1-ethenyl]-benzonitril aus Beispiel 2, 75 ml Ethanol und 75 ml 10 N Natronlauge wurden unter Rückfluß erhitzt (bis zum Ende der Umsetzung, ca. 3 h). Das Reaktionsgemisch wurde nach dem Abkuhlen auf 750 ml Eis/Wasser gegeben und mit konz. Salzsäure neutralisiert. Der entstandene Feststoff wurde abgesaugt, mit Wasser neutral gewaschen, mit Methanol nachgewaschen und im Stickstoffstrom getrocknet. Man erhielt 3,5 g der Titelverbindung, Schmp. 222—223°C (Ethanol/$H_2O$).

Die in der nachfolgenden Tabelle angegebenen Verbindungen wurden entweder durch Wittig-Horner-Reaktion (analog Beispiel 2) oder durch Verseifung der entsprechenden Nitrile (analog Beispiel 3) hergestellt:

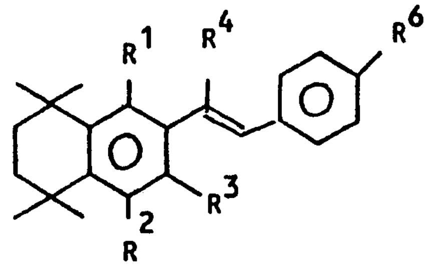

| Nr. | Name | $R^1$ | $R^3$ | $R^3$ | $R^4$ | $R^6$ | Ausbeute (%) | Schmp. (°C) |
|---|---|---|---|---|---|---|---|---|
| 4 | (E)-4-[2-(5,6,7,8-Tetrahydro-1-methoxy-4,5,5,8,8,-pentamethyl-2-naphthyl)-1-ethenyl]benzonitril | $OCH_3$ | $CH_3$ | H | H | CN | 72 | 120 |
| 5 | (E)-4-[2-(5,6,7,8-Tetrahydro-1,3-dimethoxy-5,5,8,8-tetramethyl-2-naphthyl)-1-ethenyl]benzonitril | $OCH_3$ | H | $OCH_3$ | H | CN | 99 | 168-173 |
| 6 | (E)-4-[2-(5,6,7,8-Tetrahydro-1,4-dimethoxy-5,5,8,8-tetramethyl-2-naphthyl)-1-ethenyl]benzonitril | $OCH_3$ | $OCH_3$ | H | H | CN | 70 | 154-158 |
| 7 | (e)-4-[2-(1-Ethoxy-5,6,7,8-tetrahydro-3,5,5,8,8-pentamethyl-2-naphthyl-1-ethenyl]benzonitril | $OC_2H_5$ | H | $CH_3$ | H | CN | 80 | 124-126 |
| 8 | (E)-4-[2-(5,6,7,8-Tetrahydro-3,5,5,8,8-pentamethyl-1-propyloxy-2-naphthyl)-1-ethenyl]benzonitril | $O-nC_3H_7$ | H | $CH_3$ | H | CN | 79 | 98-100 |

| Nr. | Name | $R^1$ | $R^3$ | $R^3$ | $R^4$ | $R^6$ | Ausbeute (%) | Schmp. (°C) |
|---|---|---|---|---|---|---|---|---|
| 9 | (E)-4-[2-(1-(2-Methyl)ethoxy-5,6,7,8-tetrahydro-3,5,5,8,8-pentamethyl-2-naphthyl)-1-ethenyl]benzonitril | $O-iC_3H_7$ | H | $CH_3$ | H | CN | 61 | 116-118 |
| 10 | (E)-4-[2-(1-Butyloxy-5,6,7,8-tetrahydro-3,5,5,8,8-pentamethyl-2-naphthyl)-1-ethenyl]benzonitril | $O-nC_4H_9$ | H | $CH_3$ | H | CN | 88 | 113-114 |
| 11 | (E)-4-[2-(Hexyloxy-5,6,7,8-tetrahydro-3,5,5,8,8-pentamethyl-2-naphthyl)-1-ethenyl]benzonitril | $O-nC_6H_{13}$ | H | $CH_3$ | H | CN | 46 | 90-93 |
| 12 | (E)-1-(5,6,7,8-Tetrahydro-1,3-die-methoxy-5,5,8,8-tetramethyl-2-naphthyl)-2-(4-methyl)phenylethen | $OCH_3$ | H | $OCH_3$ | H | $CH_3$ | 79 | 84-86 |
| 13 | (E)-4-[2-(5,6,7,8-Tetrahydro-1-methoxy-4,5,5,8,8-pentamethyl-2-naphthyl)-1-ethenyl]benzoesäure | $OCH_3$ | $CH_3$ | H | H | COOH | 92 | 208 (Zers.) |
| 14 | (E)-4-[2-(5,6,7,8-Tetrahydro-1,3-dimethoxy-5,5,8,8-tetramethyl-2-naphthyl)-1-ethenyl]benzoesäure | $OCH_3$ | H | $OCH_3$ | H | COOH | 51 | 212 |
| 15 | (E)-4-[2-(5,6,7,8-Tetrahydro-1,4-dimethoxy-5,5,8,8-tetramethyl-2-naphthyl)-1-ethenyl]benzoesäure | $OCH_3$ | $OCH_3$ | H | H | COOH | 100 | 80-81 |

| Nr. | Name | $R^1$ | $R^3$ | $R^3$ | $R^4$ | $R^5$ | Ausbeute (%) | Schmp. (°C) |
|---|---|---|---|---|---|---|---|---|
| 16 | (E)-4-[2-(1-Ethoxy-5,6,7,8-tetrahydro-3,5,5,8,8-pentamethyl-2-naphthyl)-1-ethenyl]benzoesäure | $OC_2H_5$ | H | $OCH_3$ | H | COOH | 94 | 144-145 |
| 17 | (E)-4-[2-(5,6,7,8-Tetrahydro-3,5,5,8,8-pentamethyl-1-propyloxy-2-naphthyl)-1-ethenyl]benzoesäure | $O-nC_3H_7$ | H | $OCH_3$ | H | COOH | 97 | 161-162 |
| 18 | (E)-4-[2-(1-(2-Methyl)ethoxy-5,6,7,8-tetrahydro-3,5,5,8,8-pentamethyl-2-naphthyl)-1-ethenyl]benzoesäure | $O-iC_3H_7$ | H | $OCH_3$ | H | COOH | 100 | 203 |
| 19 | (E)-4-[2-(1-Butyloxy-5,6,7,8-tetrahydro-3,5,5,8,8-pentamethyl-2-naphthyl)-1-ethenyl]benzoesäure | $O-nC_4H_9$ | H | $OCH_3$ | H | COOH | 99 | 210-211 |
| 20 | (E)-4-[2-(1-Hexyloxy-5,6,7,8-tetrahydro-3,5,5,8,8-pentamethyl-2-naphthyl)-1-ethenyl]benzoesäure | $O-nC_6H_{13}$ | H | $OCH_3$ | H | COOH | 97 | 133-138 |

Beispiel 21

(E)-4-[2-(5,6,7,8-Tetrahydro-1,3-dimethoxy-5,5,8,8-tetramethyl-2-naphthyl)-1-ethenyl]benzoesäureazid

Zu einer Lösung von 7,7 g (20 mmol) der Carbonsäure aus Beispiel 14 in 35 ml Aceton wurden bei 0°C zunächst 3,4 ml Triethylamin in 17 ml Aceton, dann 2,6 ml (27 mmol) Chlorameisensäureethylester zugetropft. Man ließ 40 min bei 0°C nachrühren und tropfte dann eine Lösung von 2 g (31,4 mmol) Natriumazid in 4,3 ml Wasser zu, ebenfalls bei 0°C. Es wurde noch 2 h bei 0°C gerührt, dann saugte man das Kristallisat ab und wusch mit Wasser und Ethanol nach. Nach Trocknen verblieben 6,6 g der Titelverbindung, Schmp. 125—126°C (Zers.).

Beispiel 22

(E)-4-[2-(5,6,7,8-Tetrahydro-1,3-dimethoxy-5,5,8,8-tetramethyl-2-naphthyl)-1-ethenyl]benz(2-hydroxyethyl)amid

Zu einer Lösung von 3 g (8 mmol) des in vorangegangenen Beispiel 21 beschriebenen Säureazids in 200 ml abs. THF wurden 16 ml Ethanolamin zugetropft. Nach 1,5 h Stehenlassen engte man den Ansatz teilweise ein und goß auf Wasser. Man säuerte mit 2 N HCl an, saugte den ausgefallenen Feststoff ab, wusch mehrmals mit Wasser und wenig Methanol und trocknete. Man erhielt so 2,7 g der Titelverbindung, Schmp. 201—203°C.

Beispiel 23

(E)-4-[2-(5,6,7,8-Tetrahydro-1,3-dimethoxy-5,5,8,8-tetramethyl-2-naphthyl)-1-ethenyl]benz(n-butyl)amid

Analog Beispiel 22 erhielt man aus 3 g (7,7 mmol) des in Beispiel 21 beschriebenen Säureazids und 30 ml n-Butylamin 2,3-g der Titelverbindung, Schmp. 137—140°C, nach Umkristallisation aus Methanol.

Beispiel 24

(e)-4-[2-(5,6,7,8-Tetrahydro-1,3-dimethoxy-5,5,8,8-tetramethyl-2-naphthyl)-1-ethenyl]benzamid

Ein Gemisch aus 2 g (5,3 mmol) des Nitrils aus Beispiel 5, 5 g Kaliumhydroxidpulver und 40 ml tert.-Butanol wurde 20 min. lange unter Rückfluß erhitzt. Nach dem Abkühlen goß man das Reaktionsgemisch auf gesättigte Natriumchloridlösung und extrahierte zweimal mit Ether. Der nach Trocknen ($Na_2SO_4$) und Einengen der Etherphasen erhaltene Rückstand ergab nach Umkristallisation aus Methanol 0,9 g der Titelverbindung, Schmp. 190—194°C.

Beispiel 25

(E)-4-[2-(5,6,7,8-Tetrahydro-1,3-dimethoxy-5,5,8,8-tetramethyl-2-naphthyl)-1-ethenyl]benzoesäuremethyl-ester

Zu einer Lösung von 11,8 g (30 mmol) der Carbonsäure aus Beispiel 14 in 120 ml abs. Tetrahydrofuran und 2,7 ml (56 mmol) Pyridin wurden 7,2 ml (60 mmol) Thionylchlorid in 10 ml Tetrahydrofuran zugetropft. Nach einer Reaktionszeit von 3 h fitrierte man vom Pyridinhydrochlorid ab und tropfte das Filtrat zu 10 ml abs. Methanol. Man ließ über Nacht bei Raumtemp. nachrühren, goß dann auf Wasser und extrahierte dreimal mit Ether. Die Etherphasen wurden zweimal mit Wasser gewaschen, getrocknet ($Na_2SO_4$) und eingeengt. Der Rückstand ergab nach Umkristallisation aus Methanol 5,5 g der Titelverbindung, Schmp. 140—142°C.

Beispiel 26

(E)-4-[2-(5,6,7,8-Tetrahydro-1,3-dimethoxy-5,5,8,8-tetramethyl-2-naphthyl)-1-ethenyl]benzaldehyd

Zu einer Lösung von 10,2 g (27 mmol) des Nitrils aus Beispiel 5 in 100 ml abs. Ether wurden bei Raumtemp. 47,3 ml (57 mmol) DIBAH-Lösung (20%ig in Hexan) zugegeben. Man ließ 40 min nachrühren und tropfte dann 150 ml gesättigte Weinsäurelösung zu. Nach 1 h wurde mit Ether dreimal extrahiert, die Etherphasen wurden zweimal mit Wasser gewaschen, getrocknet ($Na_2SO_4$) und eingeengt. Der Rückstand wurde zweimal aus Isopropanol umkristallisiert. Man erhielt 4,5 g der Titelverbindung, Schmp. 108°C.

Beispiel 27

(E)-4-[2-(5,6,7,8-Tetrahydro-1,3-dimethoxy-5,5,8,8-tetramethyl-2-naphthyl)-1-ethenyl]benzylalkohol

Zu einer Suspension von 1,6 g (42 mmol) Lithiumaluminiumhydrid in 200 ml abs. Ether wurde eine Suspension aus 14 g (35,5 mmol) Carbonsäure aus Beispiel 14 in 116 ml Ether zugetropft, wobei das Gemisch leicht siedete. Man rührte 3 h unter Rückfluß. Nach dem Abkühlen gab man nacheinander 50 ml Essigester, 100 ml Wasser und 150 ml 2 N HCl zu. Die Phasen wurden getrennt und die wäßrige Phase zweimal mit Ether extrahiert. Die vereinigten Etherphasen wurden neutralgewaschen, getrocknet ($Na_2SO_4$) und eingeengt. Der Rohaustrag wurde zunächst aus Heptan umkristallisiert, dann einer Blitzchromatographie (Si 60, Heptan mit steigenden Anteilen Essigester) unterworfen. Man erhielt so 6,5 g der Titelverbindung, Schmp. 102—104°C.

Beispiel 28

(E)-4-[2-(5,6,7,8-Tetrahydro-1,3-dimethoxy-5,5,8,8-tetramethyl-2-naphthyl)-1-ethenyl]benzylmethylether

Zu einer Suspension von 0,3 g (11 mmol) Nariumhydrid in 15 ml trockenem Dimethylformamid wurden 3,8 g (10 mmol) des Benzylalkoholderivats aus Beispiel 27, gelöst in 10 ml trockenem Dimethylformamid

zugetropft. Man ließ bis zum Ende der Wasserstoffentwicklung (ca. 1 h) rühren und tropfte dann unter Eiskühlung 1,56 g (11 mmol) Iodmethan zu. Man erhitzte danach 5 h bei 60°C. Anderntags tropfte man Wasser zu und saugte den ausgefallenen Feststoff ab. Dieser wurde in Methanol heiß gelöst. Beim Abkühlen auf Raumtemp. bildeten sich 2 Phasen. Die obere (Methanol)phase wurde nochmals erwärmt, dann in den Kühlschrank gestellt. Die ausgefallenen Kristalle wurden abgesaugt und getrocknet. Man erhielt so 2,1 g der Titelverbindung, Schmp. 66—68°C.

Beispiel 29

(E)-4-[2-(5,6,7,8-Tetrahydro-1,3-dimethoxy-5,5,8,8-tetramethyl-2-naphthyl)-1-ethenyl]benzylacetat

1 g (2,7 mmol) Benzylalkoholderivat aus Beispiel 27 wurden in 5 ml Pyridin gelöst und mit 1 ml Essigsäureanhydrid versetzt. Man ließ über Nach bei Raumtemp. rühren, versetzte dann mit Eis/Wasser und saugte den entstandenen Feststoff ab, der mit Waser, 0,5 N HCl und nochmals mit Wasser gewaschen wurde. Nach Trocknen blieb 1 g der Titelverbindung zurück, Schmp. 73—74°C.

Beispiel 30

(E)-4-[2-(5,6,7,8-Tetrahydro-1,3-dimethoxy-5,5,8,8-tetramethyl-2-naphthyl)-1-ethenyl]benzylamin

Zu einer Suspension von 3 g (75 mmol) Lithiumaluminiumhydrid in 150 ml abs. Ether wurden bei Raumtemp. 10 g (27 mmol) des Nitrils aus Beispiel 5, gelöst bzw. suspendiert in 170 ml Ether, zugetropft (ca. 20 min). Man erhitzte 3,5 h unter Rückfluß, hydrolysierte anderntags das abgekühlte Reaktionsgemisch mit Wasser und Natriumsulfatlösung und extrahierte die wäßrige Phase noch dreimal mit Ether. Die vereinigten Etherphasen werden mit Wasser gewaschen, getrocknet ($Na_2SO_4$) und eingeengt. Es bleiben 9,8 g der reinen Titelverbindung zurück, Schmp. 59—60°C.

Beispiel 31

N-Acetyl-(E)-4-[2-(5,6,7,8-tetrahydro-1,3-dimethoxy-5,5,8,8-tetramethyl-2-naphthyl)-1-ethenyl]benzylamin

Zu einer Lösung von 3 g (8 mmol) des Benzylaminderivats aus Beispiel 30, 1,6 g (16 mmol) Triethylamin und 50 mg DMAP (4-n.N-Dimethylaminopyridin) in 25 ml abs. Tetrahydrofuran wurde 1 ml Acetylchlorid in 5 ml abs. Tetrahydrofuran bei 20°C zugetropft. Nach 1 h bei 0°C goß man auf 100 ml Wasser und extrahierte mit Methylenchlorid. Die organische Phase wurde getrocknet und eingeengt. Der ölige Rückstand wurde mit Heptan erwärmt; nach dem Abkühlen wurde die überstehende Heptanphase abdekantiert und der Vorgang mehrmals wiederholt. Schließlich kristallisierten aus der Heptanlösung 0,8 g der Titelverbindung aus, die abgesaugt wurden, Schmp. 126—127°C.

Beispiel 32

(E,E)-4-[2-(5,6,7,8)-Tetrahydro-1,3-dimethoxy-5,5,8,8-tetramethyl-2-naphthyl)-1-ethenyl]zimtsäureethylester

Zu einer Suspension von 0,5 g (16,6 mmol) Natriumhydrid in 25 ml abs. Tetrahydrofuran wurde eine Lösung von 3,3 g (14,8 mmol) Diethylphosphonoessigsäureethylester in 15 ml abs. Tetrahydröfuran zugetropft. Man ließ 1 h nachrühren und tropfte dann eine Lösung von 2,8 g (7,4 mmol) des in Beispiel 26 beschriebenen Aldehyds in 15 ml abs. Tetrahydrofuran zu. Man ließ 16 h nachrühren, goß auf Wasser und säuerte an. Die wäßrige Phase wurde abgetrennt und zweimal mit Ether extrahiert. Die vereinigten Etherextrakte wurden einmal mit Wasser gewaschen, getrocknet ($Na_2SO_4$) und eingeengt. Der Rückstand (4,8 g) wurde aus Heptan umkristallisiert, und man erhielt 0,5 g der Titelverbindung, Schmp. 205—207°C.

**Patentansprüche für die Vertragsstaaten: BE CH DE FR GB IT LI NL SE**

1. 1-Substituierte Tetraline der Formel I

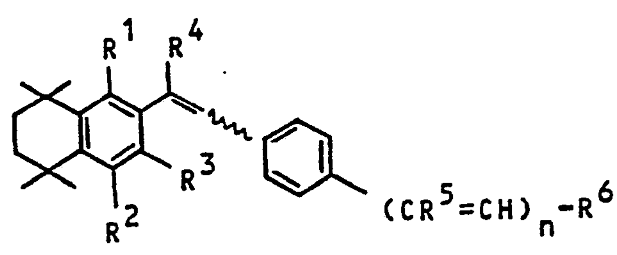

I,

in der

n die Zahl 0 oder 1,

$R^1$ eine Hydroxy- oder $C_{1-6}$-Alkoxygruppe,

$R^2$ und $R^3$ Wasserstoff- oder Halogenatome oder $C_{1-4}$-Alkyl- oder Methoxygruppen,

$R^4$ ein Wasserstoffatom oder eine gegebenenfalls cyclische Alkylgruppe mit bis zu 6 Kohlenstoffatomen,

$R^5$ ein Wasserstoffatom oder eine $C_{1-4}$-Alkylgruppe,

$R^6$ ein Wasserstoffatom, eine Nitril- oder $C_{2-10}$-Ketalgruppe, einen 2-Oxazolinyl- oder Tetrazolylrest oder den Rest —$CHR^7R^8$ oder —CO—$R^9$ bedeuten, worin

$R^7$ ein Wasserstoffatom oder eine $C_{1-3}$-Alkylgruppe

$R^8$ ein Wasserstoffatom oder den Rest —$OR^{10}$ oder —$NR^{11}R^{12}$ (mit $R^{10}$ in der Bedeutung eines Wasserstoffatoms, einer $C_{1-4}$-Alkyl-, $C_{1-20}$-Alkanoyl-, gegebenenfalls substituierten Aralkyl- oder gegebenenfalls substituierten Benzoylgruppe und $R^{11}$ und $R^{12}$ in der Bedeutung von Wasserstoffatomen, $C_{1-4}$-Alkyl-, $C_{1-20}$-Alkanoyl- oder gegebenenfalls substituierten Benzylgruppen),

$R^9$ ein Wasserstoff- oder Halogenatom, eine $C_{1-4}$-Alkylgruppe, den Azido-, Imidazol- oder Thiazolrest oder den Rest —$OR^{13}$ oder —$NR^{14}R^{15}$ darstellen (mit $R^{13}$ in der Bedeutung eines Wasserstoffatoms, einer gegebenenfalls durch Hydroxygruppen oder eine $C_{1-6}$-Alkoxygruppe substituierten $C_{1-8}$-Alkylgruppe, einer gegebenenfalls substituierten Aryl- oder Aralkylgruppe und mit $R^{14}$ und $R^{15}$ in der Bedeutung von Wasserstoffatomen, $C_{1-6}$-Alkyl- oder gegebenenfalls substituierten Aryl- oder Aralkylgruppen oder Tetrazolylreste oder $R^{14}$ und $R^{15}$ zusammen mit dem Stickstoffatom, an das sie gebunden sind, eines heterocyclischen Restes),

sowie deren physiologisch verträglichen Salze.

2. (E)-4-[2-(5,6,7,8-Tetrahydro-3,5,5,8,8-pentamethyl-1-propyloxy-2-naphthyl)-1-ethenyl]benzoesäure.

3. (E)-4-[2-(1-Butyloxy-5,6,7,8-tetrahydro-3,5,5,8,8-pentamethyl-2-naphthyl)-1-ethenyl]benzoesäure.

4. (E)-4-[2-(1-Ethoxy-5,6,7,8-tetrahydro-3,5,5,8,8-pentamethyl-2-naphthyl)-1-1-ethenyl]benzoesäure.

5. (E)-4-[2-(5,6,7,8-Tetrahydro-1,3-dimethoxy-5,5,8,8-tetramethyl-2-naphthyl)-1-ethenyl]benzoesäure.

6. 1-substituierte Tetraline de Formel I gemäß Anspruch 1 zur Verwendung bei der Bekämpfung von Krankheiten.

**Patentansprüche für den Vertragsstaat: AT**

1. Verfahren zur Herstellung der 1-substituierten Tetraline der Formel I

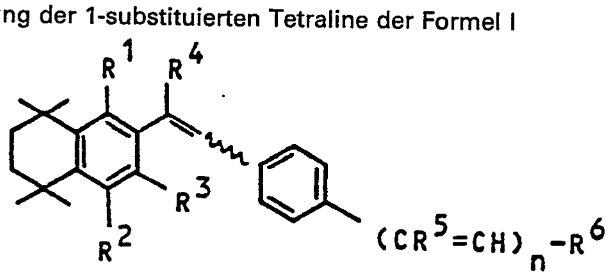

I,

in der

n die Zahl 0 oder 1,

$R^1$ eine Hydroxy- oder $C_{1-6}$-Alkoxygruppe,

$R^2$ und $R^3$ Wasserstoff- oder Halogenatome oder $C_{1-4}$-Alkyl- oder Methoxygruppen,

$R^4$ ein Wasserstoffatom oder eine gegebenenfalls cyclische Alkylgruppe mit bis zu 6 Kohlenstoffatomen,

$R^5$ ein Wasserstoffatom oder eine $C_{1-4}$-Alkylgruppe,

$R^6$ ein Wasserstoffatom, eine Nitril- oder $C_{2-10}$-Ketalgruppe, einen 2-Oxazolinyl- oder Tetrazolylrest oder den Rest —$CHR^7R^8$ oder —CO—$R^9$ bedeuten, worin

$R^7$ ein Wasserstoffatom oder eine $C_{1-3}$-Alkylgruppe

$R^8$ ein Wasserstoffatom oder den Rest —$OR^{10}$ oder —$NR^{11}R^{12}$ (mit $R^{10}$ in der Bedeutung eines Wasserstoffatoms, einer $C_{1-4}$-Alkyl-, $C_{1-20}$-Alkanoyl-, gegebenenfalls substituierten Aralkyl- oder gegebenenfalls substituierten Benzoylgruppe und $R^{11}$ und $R^{12}$ in der Bedeutung von Wasserstoffatomen, $C_{1-4}$-Alkyl-, $C_{1-20}$-Alkanoyl- oder gegebenenfalls substituierten Benzylgruppen),

$R^9$ ein Wasserstoff- oder Halogenatom, eine $C_{1-4}$-Alkylgruppe, den Azido-, Imidazol- oder Thiazolrest oder den Rest —$OR^{13}$ oder —$NR^{14}R^{15}$ darstellen (mit $R^{13}$ in der Bedeutung eines Wasserstoffatoms, einer gegebenenfalls durch Hydroxygruppen oder eine $C_{1-6}$-Alkoxygruppe substituierten $C_{1-8}$-Alkylgruppe, einer gegebenenfalls substituierten Aryl- oder Aalkylgruppe und mit $R^{14}$ und $R^{15}$ in der Bedeutung von Wasserstoffatomen, $C_{1-6}$-Alkyl- oder gegebenenfalls substituierten Aryl- oder Aralkylgruppen oder Tetrazolylreste oder $R^{14}$ und $R^{15}$ zusammen mit dem Stickstoffatom, an das sie gebunden sind, eines heterocyclischen Restes),

sowie deren physiologisch verträglichen Salze, dadurch gekennzeichnet, daß man eine Carbonylverbindung der Formel II

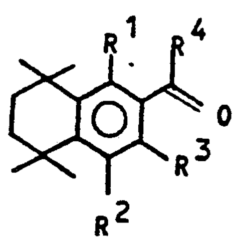

II,

in der $R^1$, $R^2$, $R^3$ und $R^4$ die angegebenen Bedeutungen haben, mit einer Phosphorverbindung der Formel III

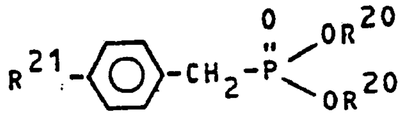 III,

in der $R^{21}$ ein Wasserstoffatom eine $C_{1-4}$-Alkylgruppe die Nitrilgruppe oder eine Gruppe —$COOR^{22}$, und $R^{20}$ und $R^{22}$ eine $C_{1-3}$-Alkylgruppe bedeutet, nach Wittig-Horner umsetzt und die so erhaltenen Verbindungen gegebenenfalls anschließend in andere Verbindungen der Formel I umwandelt.

2. Verfahren gemäß Anspruch 1, dadurch gekennzeichnet, daß man (E) - 4 - [2 - (5,6,7,8 - Tetrahydro - 3,5,5,8,8 - pentamethyl - 1 - propyloxy - 2 - naphthyl) - 1 - ethenyl]benzoesäure herstellt.

3. Verfahren gemäß Anspruch 1, dadurch gekennzeichnet, daß man (E) - 4 - [2 - (1 - Butyloxy - 5,6,7,8 - tetrahydro - 3,5,5,8,8 - pentamethyl - 2 - naphthyl) - 1 - ethenyl]benzoesäure herstellt.

4. Verfahren gemäß Anspruch 1, dadurch gekennzeichnet, daß man (E) - 4 - [2 - (1 - Ethoxy - 5,6,7,8 - tetrahydro - 3,5,5,8,8 - pentamethyl. - 2 - naphthyl) - 1 - ethenyl]benzoesäure herstellt.

5. Verfahren gemäß Anspruch 1, dadurch gekennzeichnet, daß man (E) - 4 - [2 - (5,6,7,8 - Tetrahydro - 1,3,dimethoxy - 5,5,8,8 - tetramethyl - 2 - naphthyl) - 1 - ethenyl]benzoesäure herstellt.

**Revendications pour les Etats contractants: BE CH DE FR GB IT LI NL SE**

1. Tétralines substituées en 1, de formule I

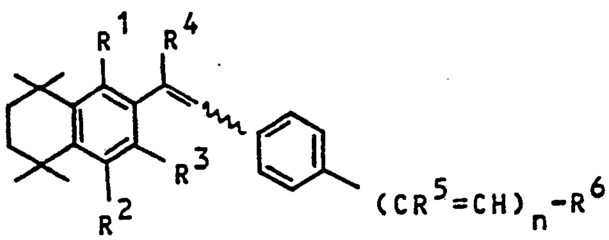 I,

dans laquelle

n représente le nombre 0 ou 1,

$R^1$, un groupe hydroxy ou alcoxy en $C_{1-6}$,

$R^2$ et $R^3$, des atomes d'hydrogène ou d'halogène ou des groupes alkyle en $C_{1-4}$ ou des groupes méthoxy,

$R^4$, un atome d'hydrogène ou un groupe alkyle, éventuellement cyclique, ayant jusqu'à 6 atomes de carbone,

$R^5$, un atome d'hydrogène ou un groupe alkyle en $C_{1-4}$,

$R^6$, un atome d'hydrogène, un groupe nitrile ou cétal en $C_{2-10}$, un reste 2-oxazolinyle ou tétrazolyle ou le reste —$CHR^7R^8$ ou —CO—$R^9$ où

$R^7$ représente un atome d'hydrogène ou un groupe alkyle en $C_{1-3}$,

$R^8$ un atome d'hydrogène ou le reste —$OR^{10}$ ou —$Nr^{11}R^{12}$ ($R^{10}$ représentant un atome d'hydrogène, un groupe alkyle en $C_{1-4}$, alcanoyle en $C_{1-20}$, aralkyle éventuellement substitué ou benzoyle éventuellement substitué, $R^{11}$ et $R^{12}$ représentant des atomes d'hydrogène, des groupes alkyle en $C_{1-4}$, alcanoyle en $C_{1-20}$ ou des groupes benzyle éventuellement substitués),

$R^9$ un atome d'hydrogène ou d'halogène, un groupe alkyle en $C_{1-4}$, le reste azido, imidazole ou thiazole ou le reste —$OR^{13}$ ou —$NR^{14}R^{15}$ ($R^{13}$ représentant un atome d'hydrogène, un groupe alkyle en $C_{1-8}$ éventuellement substitué par des groupes hydroxy ou un groupe alcoxy en $C_{1-6}$, un groupe aryle ou aralkyle éventuellement substitué, et $R^{14}$ et $R^{15}$ représentant des atomes d'hydrogène, des groupes alkyle en $C_{1-6}$, des groupes aryle ou aralkyle éventuellement substitués ou des restes tétrazolyle, ou $R^{14}$ et $R^{15}$, ensemble avec l'atome d'azote auquel ils sont liés, un reste hétérocyclique),

ainsi que leurs sels physiologiquement acceptables.

2. Acide (E) - 4 - [2 - (5,6,7,8 - tétrahydro - 3,5,5,8,8 - pentaméthyl - 1 - propyloxy - 2 - naphtyl) - 1 - éthényl]benzoïque.

3. Acide (E) - 4 - [2 - (1 - butyloxy - 5,6,7,8 - tétrahydro - 3,5,5,8,8 - pentaméthyl - 2 - naphtyl) - 1 - éthényl]benzoïque.

4. Acide (E) - 4 - [2 - (1 - éthoxy - 5,6,7,8 - tétrahydro - 3,5,5,8,8 - pentaméthyl - 2 - naphtyl) - 1 - éthényl]benzoïque.

5. Acide (E) - 4 - [2 - (5,6,7,8 - tétrahydro - 1,3 - diméthoxy - 5,5,8,8 - tetraméthyl - 2 - naphtyl) - 1 - éthényl]benzoïque.

6. Tétralines substituées en 1, de formule I selon la revendication 1, pour l'utilisation dans la lutte contre des maladies.

**Revendications pour l'Etat contractant: AT**

1. Procédé de préparation de tétralines substitués en 1, de formule I

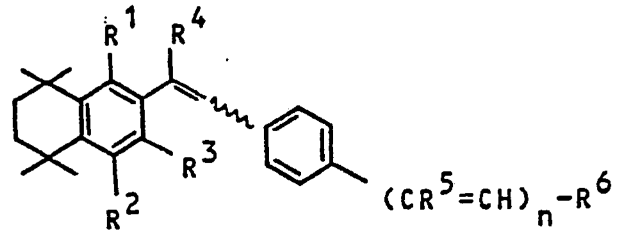

I,

dans laquelle

n représente le nombre 0 ou 1,

$R^1$, un groupe hydroxy ou alcoxy en $C_{1-6}$,

$R^2$ et $R^3$, des atomes d'hydrogène ou d'halogène ou des groupes alkyle en $C_{1-4}$ ou des groupes méthoxy,

$R^4$, un atome d'hydrogène ou un groupe alkyle, éventuellement cyclique, ayant jusqu'à 6 atomes de carbone,

$R^5$, un atome d'hydrogène ou un groupe alkyle en $C_{1-4}$,

$R^6$, un atome d'hydrogène, un groupe nitrile ou cétal en $C_{2-10}$, un reste 2-oxazolinyle ou tétrazolyle ou le reste —$CHR^7R^8$ ou —CO—$R^9$ où

$R^7$ représente un atome d'hydrogène ou un groupe alkyle en $C_{1-3}$,

$R^8$ un atome d'hydrogène ou le reste —$OR^{10}$ ou —$Nr^{11}R^{12}$ ($R^{10}$ représentant un atome d'hydrogène, un groupe alkyle en $C_{1-4}$, alcanoyle en $C_{1-20}$, aralkyle éventuellement substitué ou benzoyle éventuellement substitué, $R^{11}$ et $R^{12}$ représentant des atomes d'hydrogène, des groupes alkyle en $C_{1-4}$, alcanoyle en $C_{1-20}$ ou des groupes benzyle éventuellement substitués),

$R^9$ un atome d'hydrogène ou d'halogène, un groupe alkyle en $C_{1-4}$, le reste azido, imidazole ou thiazole ou le reste —$OR^{13}$ ou —$NR^{14}R^{15}$ ($R^{13}$ représentant un atome d'hydrogène, un groupe alkyle en $C_{1-8}$ éventuellement substitué par des groupes hydroxy ou un groupe alcoxy en $C_{1-6}$, un groupe aryle ou aralkyle éventuellement substitué, et $R^{14}$ et $R^{15}$ représentant des atomes d'hydrogène, des groupes alkyle en $C_{1-6}$, des groupes aryle ou aralkyle éventuellement substitués ou des restes tétrazolyle, ou $R^{14}$ et $R^{15}$, ensemble avec l'atome d'azote auquel ils sont liés, un reste hétérocyclique),

ainsi que leurs sels physiologiquement acceptables caractérisé par le fait qu'on fait réagir, selon Wittig-Horner, un composé carbonyle de formule II

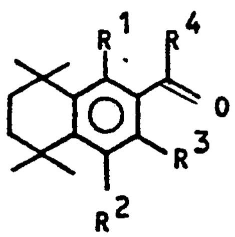

II,

dans laquelle $R^1$, $R^2$, $R^3$ et $R^4$ ont la signification indiquée, avec un composé phosphoré de formule III

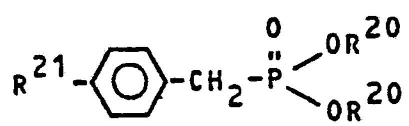

III,

dans laquelle $R^{21}$ représente un atome d'hydrogène, un groupe alkyle en $C_{1-4}$, le groupe nitrile ou un groupe —$COOR^{22}$ et $R^{20}$ et $R^{22}$ un groupe alkyle en $C_{1-3}$, et on transforme éventuellement ensuite les composés obtenus en d'autres composés de formule I.

2. Procédé selon la revendication 1, caractérisé par le fait que l'on prépare de l'acide (E)-4-[2-(5,6,7,8-tétrahydro-3,5,5,8,8-pentaméthyl-1-propyloxy-2-naphtyl)-1-éthényl]benzoïque.

3. Procédé selon la revendication 1, caractérisé par le fait que l'on prépare de l'acide (E)-4-[2-(1-butyloxy-5,6,7,8-tétrahydro-3,5,5,8,8-pentaméthyl-2-naphtyl)-1-éthényl]benzoïque.

4. Procédé selon la revendication 1, caractérisé par le fait que l'on prépare de l'acide (E)-4-[2-(1-éthoxy-5,6,7,8-tétrahydro-3,5,5,8,8-pentaméthyl-2-naphtyl)-1-éthényl]benzoïque.

5. Procédé selon la revendication 1, caractérisé par le fait que l'on prépare de l'acide (E)-4-[2-(5,6,7,8-tétrahydro-1,3-diméthoxy-5,5,8,8-tétraméthyl-2-naphtyl)-1-éthényl]benzoïque.

**Claims for the Contracting States: BE CH DE FR GB IT LI NL SE**

1. A 1-substituted tetralin of the formula I

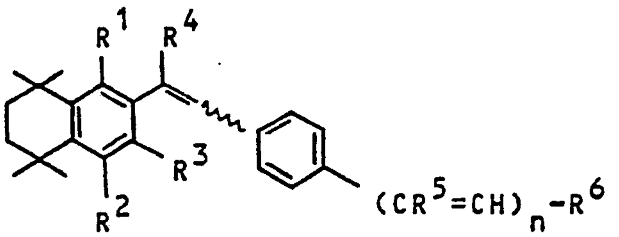

I,

where n is 0 or 1, $R^1$ is hydroxyl or $C_1$—$C_6$-alkoxy, $R^2$ and $R^3$ are each hydrogen, halogen, $C_1$—$C_4$-alkyl or methoxy, $R^4$ is hydrogen or an acyclic or cyclic alkyl group of not more than 6 carbon atoms, $R^5$ is hydrogen or $C_1$—$C_4$-alkyl, and $R^6$ is hydrogen, nitrile, $C_2$—$C_{10}$-ketal, 2-oxazolinyl, tetrazolyl or a radical —$CHR^7R^8$ or —CO—$R^9$, where $R^7$ is hydrogen or $C_1$—$C_3$-alkyl, $R^8$ is hydrogen or a radical —$OR^{10}$ or —$NR^{11}R^{12}$ (where $R^{10}$ is hydrogen, $C_1$—$C_4$-alkyl, $C_1$—$C_{20}$-alkanoyl, unsubstituted or substituted aralkyl or unsubstituted or substituted benzoyl, and $R^{11}$ and $R^{12}$ are each hydrogen, $C_1$—$C_4$-alkyl, $C_1$—$C_{20}$-alkanoyl or unsubstituted or substituted benzyl), $R^9$ is hydrogen, halogen, $C_1$—$C_4$-alkyl, azido, imidazolyl, thiazolyl or a radical —$OR^{13}$ or —$NR^{14}R^{15}$ (where $R^{13}$ is hydrogen or $C_1$—$C_8$-alkyl which is unsubstituted or substituted by hydroxyl or $C_1$—$C_6$-alkoxy, or is an unsubstituted or substituted aryl or aralkyl group, and $R^{14}$ and $R^{15}$ are each hydrogen, $C_1$—$C_6$-alkyl, an unsubstituted or substituted aryl or aralkyl group or tetrazolyl, or $R^{14}$ and $R^{15}$, together with the nitrogen atom to which they are bonded, form a heterocyclic radical), and their physiologically tolerated salts.

2. (E)-4-[2-(5,6,7,8-tetrahydro-3,5,5,8,8-pentamethyl-1-propoxynaphth-2-yl)-1-ethenyl]-benzoic acid.

3. (E)-4-[2-(1-butoxy-5,6,7,8-tetrahydro-3,5,5,8,8-pentamethylnaphth-2-yl)-1-ethenyl]-benzoic acid.

4. (E)-4-[2-(1-ethoxy-5,6,7,8-tetrahydro-3,5,5,8,8-pentamethylnaphth-2-yl)-1-ethenyl]-benzoic acid.

5. (E)-4-[2-(5,6,7,8-tetrahydro-1,3-dimethoxy-5,5,8,8-tetramethylnaphth-2-yl)-1-ethenyl]-benzoic acid.

6. A 1-substituted tetralin of the formula I as claimed in claim 1 for use in the treatment of disorders.

**Claims for the Contracting State: AT**

1. A process for the preparation of a 1-substituted tetralin of the formula I

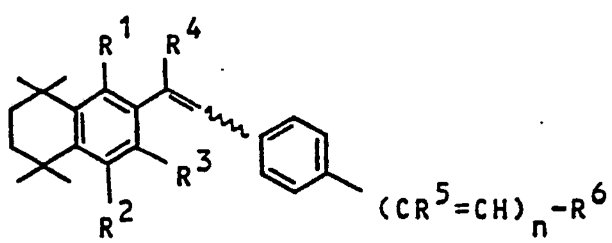

I,

where n is 0 or 1, $R^1$ is hydroxyl or $C_1$—$C_6$-alkoxy, $R^2$ and $R^3$ are each hydrogen, halogen, $C_1$—$C_4$-alkyl or methoxy, $R^4$ is hydrogen or an acyclic or cyclic alkyl group of not more than 6 carbon atoms, $R^5$ is hydrogen or $C_1$—$C_4$-alkyl, and $R^6$ is hydrogen, nitrile, $C_2$—$C_{10}$-ketal, 2-oxazolinyl, tetrazolyl or a radical —$CHR^7R^8$ or —CO—$R^9$, where $R^7$ is hydrogen or $C_1$—$C_3$-alkyl, $R^8$ is hydrogen or a radical —$OR^{10}$ or —$NR^{11}R^{12}$ (where $R^{10}$ is hydrogen, $C_1$—$C_4$-alkyl, $C_1$—$C_{20}$-alkanoyl, unsubstituted or substituted aralkyl or unsubstituted or substituted benzoyl, and $R^{11}$ and $R^{12}$ are each hydrogen, $C_1$—$C_4$-alkyl, $C_1$—$C_{20}$-alkanoyl or unsubstituted or substituted benzyl), $R^9$ is hydrogen, halogen, $C_1$—$C_4$-alkyl, azido, imidazolyl, thiazolyl or a radical —$OR^{13}$ or —$NR^{14}R^{15}$ (where $R^{13}$ is hydrogen or $C_1$—$C_8$-alkyl which is unsubstituted or substituted by hydroxyl or $C_1$—$C_6$-alkoxy, or is an unsubstituted or substituted aryl or aralkyl group, and $R^{14}$ and $R^{15}$ are each hydrogen, $C_1$—$C_6$-alkyl, an unsubstituted or substituted aryl or aralkyl group or tetrazolyl, or $R^{14}$ and $R^{15}$,

together with the nitrogen atom to which they are bonded, form a heterocyclic radical), and their physiologically tolerated salts, wherein a carbonyl compound of the formula II

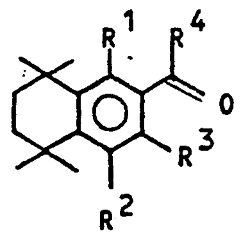

II,

where $R^1$, $R^2$, $R^3$ and $R^4$ have the stated meanings, is subjected to a Wittig-Horner reaction with a phosphorus compund of the formula III

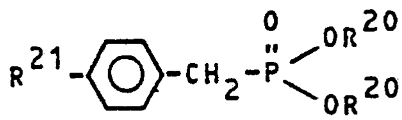

III,

where $R^{21}$ is hydrogen, $C_1$—$C_4$-alkyl, nitrile or —$COOR^{22}$, and $R^{20}$ and $R^{22}$ are each $C_1$—$C_3$-alkyl, and the compounds obtained in this manner may subsequently be converted into other compounds of the formula I.

2. A process as claimed in claim 1, wherein (E)-4-[2-(5,6,7,8-tetrahydro-3,5,5,8,8-pentamethyl-1-propoxynaphth-2-yl)-1-ethenyl]-benzoic acid is prepared.

3. A process as claimed in claim 1, wherein (E)-4-[2-(1-butoxy-5,6,7,8-tetrahydro-3,5,5,8,8-pentamethylnaphth-2-yl)-1-ethenyl]-benzoic acid is prepared.

4. A process as claimed in claim 1, wherein (E)-4-[2-(1-ethoxy-5,6,7,8-tetrahydro-3,5,5,8,8-pentamethylnaphth-2-yl)-1-ethenyl]-benzoic acid is prepared.

5. A process as claimed in claim 1, wherein (E)-4-[2-(5,6,7,8-tetrahydro-1,3-dimethoxy-5,5,8,8-tetramethylnaphth-2-yl)-1-ethenyl]-benzoic acid is prepared.